# EUROPÄISCHE PATENTANMELDUNG

(11) **EP 2 388 125 A2**
(43) Veröffentlichungstag der Anmeldung: **23.11.2011**
(21) Anmeldenummer: 11164040.5
(22) Anmeldetag: 28.04.2011
(51) Int. Cl.: B29C 49/42, B29C 49/46

(54) **Sterilisierbare Blasform**

(30) Priorität: 20.05.2010 DE 102010022131
(71) Anmelder: Krones AG, 93073 Neutraubling (DE)
(72) Erfinder: Voth, Klaus, 93083, Obertraubling (DE); Martini, Oliver, 3510, Konolfingen (CH); Lappe, Ulrich, 93059, Regensburg (DE); Söllner, Jürgen, 93176, Beratzhausen (DE); Winzinger, Frank, 93049, Regensburg (DE); Hausladen, Josef, 93086, Wörth/Donau (DE)
(74) Vertreter: Hannke, Christian

(57) **Zusammenfassung**

Die Erfindung betrifft ein Verfahren zum Behandeln von Einrichtungen einer Anlage zum Herstellen von Kunststoffbehältnissen, mit den Schritten:
- Auswählen mindestens einer Behandlungseinrichtung (1), Beaufschlagen der Behandlungseinrichtung (1) mit einer Sterilisiertemperatur zum Sterilisieren der Behandlungseinrichtung (1),
- Zuführen von mindestens einem Kunststoffvorformling zu der Behandlungseinrichtung (1),
- Umformen des Kunststoffvorformlings zu einem Kunststoffbehältnis,
- Abführen des mindestens einen Kunststoffbehältnisses aus der Behandlungseinrichtung (1).

## Beschreibung

Die vorliegende Erfindung ist auf ein Verfahren zum Sterilisieren einer Behandlungseinrichtung in einer Anlage zum Herstellen von Kunststoffbehältnissen gemäß Anspruch 1, eine Blasform zum Umformen von Kunststoffvorformlingen in Behältnisse gemäß dem Oberbegriff des Patentanspruchs 9 und auf eine Verwendung eines Temperierungsmittels zum Temperieren einer Blasform gemäß Anspruch 10 gerichtet. Blasformen gemäß der vorliegenden Erfindung werden besonders bevorzugt zum Umformen von Preforms bzw. Kunststoffvorformlingen in Behältnisse, wie beispielsweise PET-Flaschen, Kanister, Fässer und ähnlichem verwendet.

Gemäß modernen Anforderungen an die Keimfreiheit von Abfüllanlagen, insbesondere aseptische Behandlungsanlagen, ist es erforderlich die Behältnisse möglichst während dem gesamten Behandlungsprozess in einer keimfreien Umgebung vorzusehen. Es ist somit nicht nur erforderlich das Innere der Behältnisse bzw. der Preforms steril bzw. keimfrei zu halten, sondern ebenfalls das Äußere der Preforms bzw. Behältnisse steril bzw. keimfrei zu halten, damit eine Übertragung von sich auf der Außenseite der Behältnisse befindender Bakterien, Viren, Keime oder ähnlichem nicht in dem Bereich des Innenraums der Behältnisse bzw. Preforms erfolgen kann und somit ein Kontakt des Füllguts mit Bakterien, Viren, Keime und/oder ähnlichem verhindert wird.

Es ist somit Aufgabe der vorliegenden Erfindung, sicherzustellen, dass auch während der Umformung eines Preforms zu einem Behältnis möglichst wenige Keime, Bakterien, Viren oder ähnliches in den Innenraum oder in den Bereich des Mundstückes eines Behältnisses beim Umformen eines Kunststoffvorformlings in ein Behältnis eindringen können.

Erfindungsgemäß wird die zuvor gestellte Aufgabe durch ein Verfahren zum Behandeln von Einrichtungen einer Anlage zum Herstellen von Kunststoffbehältnissen, das die nachfolgenden Schritte beinhaltet, gelöst. Besonders bevorzugt handelt es sich bei der besagten Anlage um eine Anlage, die mindestens einen Reinraum aufweist.

Die nachfolgend angeführten Verfahrensschritte werden bevorzugt in genau der nachfolgend genannten Abfolge durchgeführt. Wobei ebenfalls denkbar ist, dass Abweichungen von dem nachfolgend wiedergegebenen Ablauf möglich sind oder einzelne Verfahrensschritte sich überlappen.

So erfolgt in einem ersten Schritt bevorzugt das Auswählen mindestens einer Behandlungseinrichtung, die insbesondere eine Blasform ist, die besonders bevorzugt zumindest zeitweise in einem Reinraum angeordnet ist und die bevorzugt zumindest zeitweise das Kunststoffbehältnis zumindest teilweise umschließt bzw. geeignet ist einen Kunststoffvorformling und/oder ein Behältnis aufzunehmen oder zu umschließen.

Unter Umschließen wird insbesondere auch ein kurzzeitiges Umrahmen oder Einfassen des Kunststoffvorformlings, teilweise mit und teilweise ohne direkte Berührung des Vorformlings, durch eine oder mehrere Behandlungseinrichtungen verstanden.

In einem zweiten Schritt erfolgt bevorzugt das Beaufschlagen der Behandlungseinrichtung mit einer Sterilisiertemperatur zum Sterilisieren der Behandlungseinrichtung. Anschließend erfolgt bevorzugt eine Einstellung einer Bearbeitungstemperatur die während den Bearbeitungsschritten, d. h. dem Umformen einer Vielzahl an Preforms in Kunststoffbehältnisse, insbesondere in Kunststoffflaschen, erforderlich ist.

Nach dem optionalen Schritt des Einstellens einer Bearbeitungstemperatur erfolgt bevorzugt die Zuführung von mindestens einem Kunststoffvorformling zu der Behandlungseinrichtung, in der in einem weiteren Schritt die Umformung des Kunststoffvorformlings zu einem Kunststoffbehältnis durchgeführt wird. Nach der abgeschlossenen Umformung wird das Kunststoffbehältnis besonders bevorzugt aus der Behandlungseinrichtung abgeführt.

In dem Reinraum herrscht besonders bevorzugt ein Druck, der höher ist als der Umgebungsdruck. Ferner ist die Behandlungseinrichtung bevorzugt zwischen einer Transporteinrichtung für Preforms und einem Füller angeordnet. Vorteilhaft ist mindestens eine Transporteinrichtung zum Transportieren der Preforms und/oder Behältnisse im Reinraum angeordnet.

Die vorliegende Erfindung ist vorteilhaft, da eine Sterilisierung der Anlage vor der Durchführung von Arbeitsschritten, insbesondere dem Umformen von Preforms bzw. dem Erzeugen von Behältnissen, möglich ist. Dadurch ist eine Keimreduktion bis hin zur absoluten Keimfreiheit bei der Gesamtheit der in einer entsprechenden Blasmaschine verbauten Blasstationen bzw. Blasformen erzielbar.

Bevorzugt wird nach Beendigung der Arbeitsschritte bzw. beim Produktionsende und/oder während Produktionspausen die Anlage, insbesondere die Behandlungseinrichtung, getrocknet. Dies kann beispielsweise erforderlich sein, wenn die Blasstation bzw. die Blasform während des Produktionsbetriebes gekühlt (d. h. beispielsweise unter Raumtemperatur) betrieben wird, da dies je nach Umgebungsbedingungen zu Niederschlag führt und Kondenswasser in der jeweiligen Blasstation bzw. Blasform entsteht. Es ist bekannt, dass an Orten mit Nässe ein hohes Risiko von Keimpräsenz und Keim-Vermehrung vorliegt. Bevorzugt ist daher die Entstehung und/oder das Ansammeln von Kondenswasser während des Produktionsbetriebes der Steckblasstation zu vermeiden. Dies erfolgt bevorzugt durch eine Temperierung der Behandlungseinrichtung, insbesondere durch eine Aufheizung auf eine Temperatur, bei der die Flüssigkeit, wie z. B. die Kondensflüssigkeit, in der Behandlungsanlage verdampft. Bei Wasser ist dies bei 1 bar z. B. bei einer Temperatur von >100°C möglich, somit wird ein Abdampfen des Kondenswassers physikalisch sichergestellt.

In einer weiteren bevorzugten Ausführungsform der vorliegenden Erfindung ist mindestens ein bevorzugt permanent mit der Behandlungseinrichtung in einer thermischen Verbindung stehendes Temperierungsmittel vorgesehen, durch das die Behandlungseinrichtung auf die Sterilisiertemperatur temperiert wird bzw. temperierbar ist.

Das Temperierungsmittel kann dabei beispielsweise als elektrische Heizung ausgebildet sein, die beispielsweise Heizspiralen aufweist. Es ist ebenfalls denkbar, dass die einzelnen Blasformkörper Heizwiderstände ausbilden, die sich beim Leiten von Elektrizität erwärmen. In diesem Fall ist das Temperierungsmittel durch die Kontakte bzw. die elektrischen Anschlüsse ausgebildet. Die Temperatur wird bevorzugt zumindest auf der Oberfläche der Blasform und besonders bevorzugt der inneren Oberfläche der Blasform zugeführt. Dies erfolgt beispielsweise mittels Wärmeleitung und/oder Wärmestrahlung.

Es ist somit ebenfalls denkbar, dass ein bzw. mehrere Heizstrahler in die Blasformhälften bzw. zwischen die Blasformhälften einbringbar sind. Derartige Heizstrahler können beispielsweise in Form von Platten oder stabförmigen Heizstrahlern ausgebildet sein. Es ist ferner denkbar, dass Heizstrahler vorgesehen sind, um die Blasform bzw. die Blasformteile im offenen oder geschlossenen Zustand mit Wärmestrahlung zu beaufschlagen. Die Blasformhälften können somit im geöffneten Zustand zwischen Heizstrahlern angeordnet sein und mit Wärme beaufschlagt werden.

Weiterhin ist vorstellbar, dass ein Wärmeträgerfluid über einen bevorzugt geschlossenen Kreislauf die Blasstation bzw. die Blasform oder einen der Blasformteile auf eine vorgegebene Temperatur bringt. Das Wärmeträgerfluid wird den einzelnen Blasformteilen bevorzugt über ein Kanalsystem zugeführt.

Diese Ausführungsform ist vorteilhaft, da beispielsweise die benötigte Sterilisiertemperatur, die Geschwindigkeit der Erhitzung, die Erhitzungsdauer und/oder ähnliches einstellbar bzw. regelbar ist.

Allgemein wird mit der Formulierung Temperierung eine Zuführung von Wärme, insbesondere zum Aufheizen der Blasform bzw. der Blasformteile verstanden.

In einer weiteren bevorzugten Ausführungsform der vorliegenden Erfindung wird die Blasform von einem durch das Temperierungsmittel temperierten Fluid zumindest abschnittsweise um- und/oder durchströmt. Es ist hierbei denkbar, dass ein Kanal bzw. eine Vielzahl an Kanäle innerhalb eines bzw. beider Blasformteile ausgebildet ist. Ferner ist vorstellbar, dass eine Fluidkommunikation von einem Blasformteil zu dem anderen Blasformteil bzw. einem weiteren Blasformteil möglich ist. Somit ist z. B. beiden Blasformteilen das Fluid zu- und abführbar oder lediglich einem Blasformteil zuführbar und von dem anderen abführbar oder das Fluid kann einem Blasformteil zugeführt werden und von diesem abgeführt werden, wobei das Fluid durch beide bzw. mehrere (oder alle) Blasformteile geführt wird bzw. führbar ist. Es ist somit ersichtlich, dass verschiedene Blasformteile bevorzugt gleich oder verschieden beheizbar sind.

Zur einfachen Verteilung der Wärme in allen Bauteilen, die sterilisiert werden sollen, sind diese Bauteile wegen der guten Wärmeleitung bevorzugt aus Metall gefertigt, wobei Aluminiumwerkstoffe wegen ihrer hohen Wärmeleitkoeffizienten bevorzugt sind. Es ist jedoch ebenfalls denkbar, dass Stahl- und Edelstahlwerkstoffe oder ähnliche Werkstoffe verwendet werden können. Alternativ ist auch eine Kombination aus verschiedenen Werkstoffen, wie z.B. Aluminium verstärkt mit einem oder mehreren weiteren Metallen, bei diesen Bauteilen denkbar.

Bevorzugt wird durch das Temperierungsmittel ein Fluid der Blasform zugeführt, das bereits als Arbeitsfluid, d. h. bevorzugt zum Aufheizen, Betreiben und/oder Abkühlen weiterer Einrichtungen der Anlage verwendet wurde bzw. nachfolgend verwendet wird.

Eine alternative Möglichkeit, die beschriebene Erhitzung der Blasstation bzw. der Blasform zu realisieren ist die Nutzung eines zusätzlichen nur für diese Erhitzung vorzusehenden Kanalsystems, das erlaubt andere als die für die Temperierung bei der Produktion bzw. während dem Arbeitsschritt vorgesehene Systeme (wie z. B. Heizeinrichtungen) und Fluide zu nutzen. Besonders geeignet ist beispielsweise die Zuführung von Heißwasser, überhitztem Wasser oder Wasserdampf. In bestimmten Fällen ist es ebenfalls denkbar ein einziges Kanalsystem umschaltbar mit den verschiedenen Fluiden für die Produktion und Sterilisation zu betreiben.

Diese Ausführungsform ist vorteilhaft, da die Blasform bzw. die Blasformteile definiert erwärmbar sind und die Abwärme weiterer zu der Anlage gehörender Einrichtungen und/oder eine spezielle Erhitzung mittels eines Temperierungsmittels möglich ist.

In einer weiteren bevorzugten Ausführungsform der vorliegenden Erfindung besteht das Fluid zumindest teilweise aus einer Wasser-Glykol-Mischung, Thermoöl, Wasser und/oder ähnlichen. Es ist jedoch ebenfalls denkbar, dass, wie bereits zuvor angeführt, ein dampfförmiges Medium, wie beispielsweise Wasserdampf oder überhitzter Wasserdampf als Fluid verwendet wird.

Diese Ausführungsform ist vorteilhaft, da eine Vielzahl verschiedener Fluide zum Übertragen von Wärme auf die Blasform bzw. die Blasformteile verwendbar ist. Dadurch können je nach Erfordernissen jeweils hinsichtlich der Kosten, des Prozessverlaufs, ökologischer Aspekte oder ähnlichem die geeignetsten Fluide ausgewählt werden.

In einer weiteren bevorzugten Ausführungsform der vorliegenden Erfindung wird die Behandlungseinrichtung auf eine Sterilisiertemperatur von mindestens 60°C, bevorzugt 100°C und besonders bevorzugt mehr als 121°C temperiert, wobei die Sterilisiertemperatur bevorzugt durch elektrische Erwärmung und/oder durch elektromagnetische Erwärmung, insbesondere mittels Induktion, erreicht wird bzw. erreichbar ist. Besonders bevorzugt wird die Sterilisiertemperatur somit durch mindestens ein der Blasform zugeordnetes Temperiermittel oder eine Heizeinrichtung, wie z.B. mindestens eine Heizspirale und/oder mindestens eine Spule erzeugt.

Die zuvor genannten Temperaturen sind bevorzugt in einem Druckbereich des Mediums bzw. der Umgebung der Blasform von 0,2 bar bis 50 bar und besonders bevorzugt bei einem Druck zwischen ca. 1 bar und ca. 15 bar vorgesehen. Wobei auch denkbar ist, dass in Abhängigkeit von den gegebenen Drücken die Sterilisiertemperatur verschiedene bzw. an den jeweiligen Druck angepasste Werte umfasst. Dabei ist beispielsweise denkbar, dass im Falle niedriger Drücke eine Sterilisierung der Blasform ebenfalls bei einer niedrigeren Temperatur durchführbar ist.

In einer weiteren bevorzugten Ausführungsform der vorliegenden Erfindung wird die Behandlungseinrichtung mindestens 10 Minuten, bevorzugt mindestens 20 Minuten und besonders bevorzugt mindestens 30 Minuten temperiert. Es ist hierbei jedoch auch denkbar, dass die Behandlungseinrichtung wesentlich kürzer, d. h. beispielsweise eine Minute oder drei Minuten temperiert wird.

Bevorzugt beschreibt die zuvor genannte Zeitphase jeweils die Zeit zwischen dem Schließen und dem Öffnen der Blasform, wobei auch denkbar ist, dass lediglich die Zeit, in der eine aktive Erhitzung der Blasform erfolgt, als Temperierungszeit angesehen wird.

Eine Keimreduktion bis hin zur absoluten Keimfreiheit erfolgt bevorzugt an einer bzw. an der Gesamtheit der in der entsprechenden Blasmaschine verbauten Blasstationen bzw. Blasformen und somit bevorzugt durch eine Erhitzung der Station bzw. Blasform vor dem Produktionsbetrieb auf die bereits genannten Temperaturen. Im Falle eines geringeren Anspruchs an die Keimreduktion kann ein wirksamer Effekt schon ab einer Temperatur von ca. 50°C oder ≥ 60°C erreicht werden.

In einer weiteren bevorzugten Ausführungsform der vorliegenden Erfindung wird die Behandlungseinrichtung nach dem Sterilisieren der Blasform zum Zuführen der Behältnisse geöffnet. Somit ist die Blasform bevorzugt während des Sterilisierens geschlossen, es ist jedoch ebenfalls vorstellbar, dass die Blasform während des Sterilisierens teilweise oder vollständig geöffnet ist. Besonders bevorzugt ist während des Sterilisierens bzw. in der Sterilisationsphase die Reckstange in die Blasform bzw. in den durch die Blasformteile gebildeten Hohlraum eingeführt oder verschließt diesen bevorzugt zumindest teilweise, wobei besonders bevorzugt während dem Sterilisieren kein Preform oder Behältnis in der Behandlungseinrichtung angeordnet ist.

Diese Ausführungsform ist vorteilhaft, da die Sterilisation bevorzugt bei einer zumindest teilweise geschlossenen oder vollständig geschlossenen Blasform erfolgt, wodurch die Hitze im Bereich der inneren Oberfläche der Blasform gestaut wird und eine Temperierung effizient durchführbar ist.

In einer weiteren bevorzugten Ausführungsform der vorliegenden Erfindung wird der Behandlungseinrichtung vor und/oder während der Beaufschlagung mit der Sterilisiertemperatur mindestens ein Desinfektionsmittel zugeführt, insbesondere der inneren Oberfläche der Blasform. Es ist hierbei vorstellbar, dass in der Umgebung der Blasform bzw. in den einzelnen Blasformteilen Zuführeinrichtungen ausgebildet sind, mittels denen das Infektionsmittel flüssig und/oder gasförmig zuführbar ist. Das Desinfektionsmittel kann dabei erhitzt, d. h. in einer Temperatur größer oder gleich der Sterilisationstemperatur, oder kalt, d. h. in einer Temperatur unter der Sterilisationstemperatur, zugeführt werden. Bevorzugt wird das Desinfektionsmittel automatisch bevorzugt in Abhängigkeit definierter Zeit-, Temperaturwerte oder ähnlichem zugeführt. Es ist jedoch ebenfalls denkbar, dass das Desinfektionsmittel mittels einer Spendereinrichtung und somit manuell der Behandlungseinrichtung zugeführt wird bzw. zuführbar ist.

Diese Ausführungsform ist vorteilhaft, da speziell durch die Verdampfung des Desinfektionsmittels während dem Aufheizvorgang bzw. während der Temperierung der Blasform die Verdampfung des Desinfektionsmittels und/oder der Kontakt des Desinfektionsmittels mit den Keimen die Keimreduktion besonders unterstützt.

Die vorliegende Erfindung ist ebenfalls auf eine Blasform zum Umformen von Vorformlingen in Behältnisse gerichtet. Die Blasform umfasst mindestens einen ersten Teil einer Blasformwandung und einen gegenüber dem ersten Teil bewegbaren zweiten Teil einer Blasformwandung mit jeweils einer inneren und einer äußeren Oberfläche, wobei durch die innere Oberfläche zumindest abschnittsweise eine negative Behälterform ausgebildet ist und mittels einer Kopplungseinrichtung die Teile der Blasformwandung zum Ausbilden eines geschlossenen Zustands miteinander koppelbar sind. Erfindungsgemäß ist mindestens im Bereich eines Teils der Blasformwandung zumindest teilweise ein Temperierungsmittel zum Temperieren der Blasform mit einer Sterilisiertemperatur angeordnet.

Bevorzugt ist ferner eine Steuereinrichtung vorgesehen, die derart gestaltet ist, dass eine Sterilisierung bzw. die Temperaturbeaufschlagung der Blasform im Wesentlichen außerhalb und bevorzugt komplett außerhalb des Arbeitsbetriebs erfolgt. Der Arbeitsbetrieb beschreibt bevorzugt die Phase, in der eine Umformung mehrerer Preforms in Behältnisse nacheinander mit derselben Blasform erfolgt, wobei vorstellbar ist, dass eine Vielzahl an Blasformen parallel und/oder nacheinander verwendet wird. Es ist somit bevorzugt eine Steuerungsvorrichtung vorhanden, die zum Steuern des Temperiermittels zum Temperieren der Blasform ausgebildet ist, wobei die Blasform außerhalb des eigentlichen Betriebs auf eine bestimmte Sterilisiertemperatur gebracht wird bzw. bringbar ist.

Bevorzugt wird mit der Steuereinrichtung und/oder einer weiteren Datenverarbeitungs- oder Eingabeeinheit zumindest eine Blasform, eine Blasdüse, eine Reckstange, eine Bodenform und/oder eine auf dem Formträger angeordnete Klammer zum Übernehmen des Vorformlings aus einer Transfereinrichtung für die anschließende Sterilisierung als Behandlungseinrichtung ausgewählt.

Weiterhin ist die vorliegende Erfindung auf eine Verwendung eines Temperierungsmittels zum Temperieren einer Blasform einer Behälterbehandlungsanlage mit einer Sterilisiertemperatur gerichtet.

An dieser Stelle sei noch darauf hingewiesen, dass sämtliche in den Anmeldeunterlagen offenbarten Merkmale einzeln oder in Kombination miteinander gattungsgemäße oder aus dem Stand der Technik bekannte Blasformen vorteilhaft weiterentwickeln.

Weitere Vorteile, Ziele und Eigenschaften der vorliegenden Erfindung werden anhand nachfolgender Beschreibung anliegender Zeichnungen erläutert, in welchen beispielhaft Blasformen für die Umformung von Kunststoffvorformlingen in Behältnisse dargestellt sind. Bauteile der Blasformen, welche in den Figuren wenigstens im Wesentlichen hinsichtlich ihrer Funktion übereinstimmen, können hierbei mit gleichen Bezugszeichen gekennzeichnet sein, wobei diese Bauteile nicht in allen Figuren beziffert oder erläutert sein müssen.

Darin zeigen:
- Fig. 1: eine Blasform mit einer Temperierungseinrichtung in einem ersten geschlossenen Zustand;
- Fig. 2: eine Blasform mit einer Temperierungseinrichtung in einem zweiten geöffneten Zustand;
- Fig. 3: eine schematische Darstellung einer Anbindung eines oder mehrerer Temperiermittel an eine Blasstation; und
- Fig. 4: eine zweidimensionale Darstellung einer Rotationsblasstation.

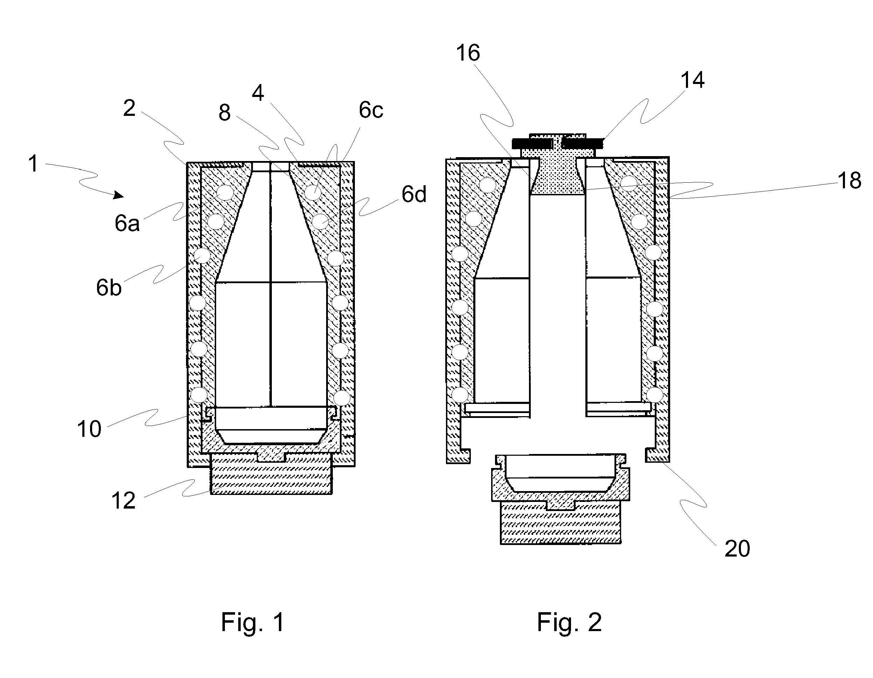

Fig. 1 zeigt eine Blasform 1, die aus einem ersten Blasformteil 2 und einem zweiten Blasformteil 4 besteht. Auf einer Seite wird die Blasform 1 von einem Boden- und/oder Deckelelement 12abgeschlossen. Die Blasform 1 weist in dem ersten Blasformteil 2 und dem zweiten Blasformteil 4 jeweils Temperierungsmittel 6a-6d auf. Die Temperierungsmittel 6a-6d können hierbei in einer beliebigen Vielzahl vorgesehen sein oder nur ein bzw. jeweils nur ein Temperierungsmittel ausbilden und jeweils miteinander funktional in Verbindung stehen. Besonders bevorzugt ist ein Fluidaustausch zwischen den Temperierungsmitteln 6a, 6b oder 6a, d oder 6a, 6c möglich. Die Temperierungsmittel 6a-6d sind bevorzugt in einem Wandungsbereich zwischen der inneren Oberfläche 8 und der äußeren Oberfläche 10 der Blasform 1 angeordnet. Es ist zudem denkbar, dass die Temperierungsmittel 6a-6d ebenfalls abschnittsweise an bzw. auf der äußeren Oberfläche der Blasform 1 bzw. eines oder beider Blasformteile 2, verlaufen. Selbstverständlich ist ebenfalls vorstellbar, dass die Temperierungsmittel 6a-6d am Boden-/Deckelelement 12 angeordnet bzw. vorgesehen sind. Bevorzugt sind die Temperierungsmittel 6a-6d als elektrische Widerstandheizung und besonders bevorzugt als eine mindestens einen Kanal aufweisende Fluidheizung ausgeführt, wobei die mit den Bezugszeichen 6a-6d gekennzeichneten Elemente in einer Querschnittdarstellung dargestellte Kanäle bzw. Kanalabschnitte eines oder mehrerer Kanäle sind. Denkbar ist auch eine Blasdüse, eine Reckstange und/oder eine auf dem Formträger angeordnete Klammer zum Übernehmen des Vorformlings aus einer Transfereinrichtung bei einer derartigen Sterilisierung mit einzubeziehen, dies kann beispielsweise bedeuten, dass einzelne oder alle der genannten Elemente nicht, teilweise oder vollständig sterilisierbar sind.

In Fig. 2 ist gegensätzlich zur Fig. 1 kein geschlossener Zustand, sondern ein geöffneter Zustand der Blasform 1 dargestellt. Ferner ist dieser Darstellung zu entnehmen, dass eine Positioniereinrichtung 14, wie z. B. eine Greifklammer, vorgesehen ist, um ein Halteelement 18, an dem ein Eingriffelement 16 ausgebildet ist, in den Innenraum der Blasform 1 bzw. zwischen den ersten Blasformteil 2 und den zweiten Blasformteil 4 zu bewegen. Mit dem Bezugszeichen 20 ist eine Kopplungseinrichtung 20 gekennzeichnet. Die Kopplungseinrichtung 20 kann derart ausgebildet sein, dass die Blasformteile 2, 4 z.B. formschlüssig, feldschlüssig und/oder reibschlüssig in einem geschlossenen Zustand miteinander gekoppelt bzw. koppelbar sind.

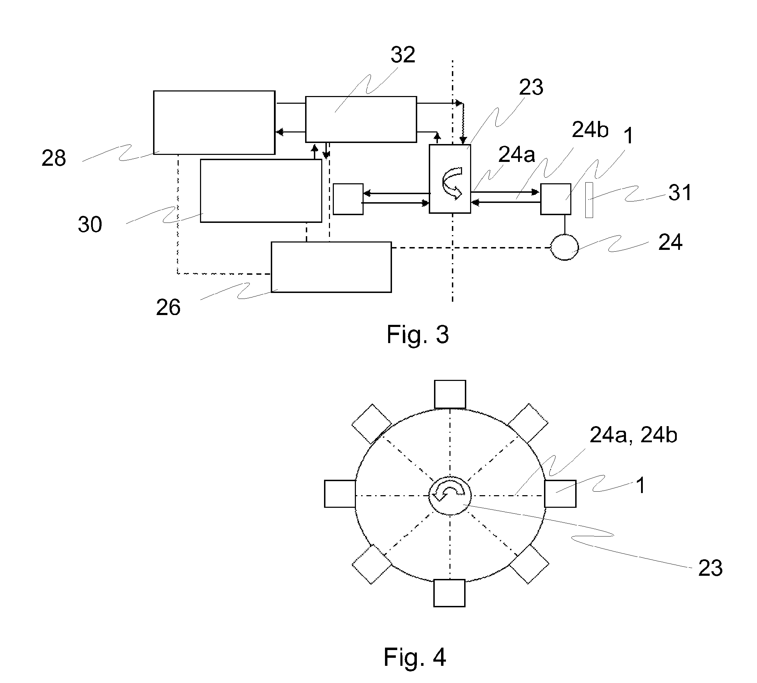

In Fig. 3 ist eine schematische Darstellung möglicher Kommunikationswege im Rahmen einer Temperierung der Blasform 1 zwischen benötigten und/oder optionalen Einrichtungen zu entnehmen. Nicht beschriebene aber dargestellte Kommunikationswege beschreiben bevorzugt einen Fluidtransfer, einen Daten- und/oder Signalaustausch, einen Energieaustausch oder ähnliches, wobei die Kommunikation jeweils in beide Richtungen oder nur in eine Richtung erfolgen kann.

Die Blasformen 1 stehen mit einem Mediendrehverteiler 23 über Kommunikationswege 24a, 24b kommunizierend in Verbindung. Die Kommunikationswege 24a, 24b sind erforderlich, sobald eine Temperierung der Blasform 1 mittels mindestens einem Funktionsfluid bzw. Wärmetransportfluid erfolgt, da das temperierte Fluid der Blasform 1 zuführbar sein muss. Es ist jedoch denkbar, dass zusätzliche oder alternative Kommunikationswege vorgesehen sein können.

Es ist ferner denkbar, dass im Bereich der jeweiligen Blasform 1 elektrische oder optische Heizeinrichtungen zum Temperieren der Blasformen 1 vorgesehen bzw. vorsehbar sind. So ist es beispielsweise vorstellbar, dass alternativ bzw. optional zu dem Einsatz fluidischer Temperiermittel 28, 30 eine Temperierung mittels einer Widerstandsheizung 31 oder Bestrahlung 31, insbesondere eine Infrarot- und/oder UV-Bestrahlung, erfolgt.

Mit dem Bezugszeichen 24 ist ein Sensor gekennzeichnet, der optional vorgesehen werden kann und mittels dem beispielsweise eine Überprüfung von Temperatursollwerten und/oder der Temperierungsdauer und/oder eine Überwachung der Kontamination durchführbar ist. Der Sensor 24 steht besonders bevorzugt mit einer Maschinensteuerung 26 kommunizierend bzw. Daten oder Signale austauschend in Verbindung. Die Maschinensteuerung 26 dient besonders bevorzugt zur Steuerung von mindestens einem Temperiermittel 28, das bevorzugt Arbeitsfluide weiterer Einrichtungen, die bevorzugt mindestens die gewünschte Sterilisiertemperatur aufweisen, zu dem Mediendrehverteiler 23 fördert. Es sei hierbei angemerkt, dass anstelle eines Mediendrehverteilers 23 ebenfalls ein Linearverteiler vorgesehen sein kann.

Durch das Bezugszeichen 30 ist ein spezielles Temperiermittel gekennzeichnet, das alternativ oder optional zu dem Temperiermittel 28 und/oder zu einer elektrischen bzw. optischen Heizung bzw. Temperierung vorsehbar ist. Im Falle zweier Temperiermittel 28, 30 oder noch weiterer Temperiermittel 31 ist besonders bevorzugt eine Regeleinheit, insbesondere eine Umschaltventilgruppe 32, vorgesehen, die beispielsweise zeitgleich durch die Temperiermittel 28, 30 temperierte Fluide den Blasformen 1 zu- oder abführt. Es ist ebenfalls vorstellbar, dass durch die Umschaltventilgruppe 32 lediglich ein mittels einem der Temperiermittel 28, 30 temperiertes Fluid der Blasform 1 zuführbar ist. Die Umschaltventilgruppe 32 kann daher beispielsweise die Zuführung eines Fluids zu einem Kanal 6a-6d, der zu der Blasform 1 führt, bewirken bzw. einstellen. Die Regeleinheit 32 kann manuell oder automatisch betätigt werden, bevorzugt erfolgt eine automatische Betätigung jedoch in Abhängigkeit von bestimmten Betriebsparametern, wie z.B. der Fluidtemperatur, der Temperatur der Blasform 1, der Transportgeschwindigkeit der Blasform 1, der Temperierungsdauer und/oder ähnlichen.

In Fig. 4 ist eine zweidimensionale Darstellung des Mediendrehverteilers 23 dargestellt. Es ist dieser Darstellung zu entnehmen, dass der Mediendrehverteiler 23 mit einer Vielzahl an Blasformen 1 ausgestattet sein kann, die jeweils bevorzugt über mindestens eine Zuführleitung 24a und eine Abführleitung 24b mit einem Arbeitsfluid bzw. einem Wärmeübertragungsfluid versorgt werden können.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Blasform
- 2: erster Blasformteil
- 4: zweiter Blasformteil
- 6a-6d: Temperierungsmittel
- 8: innere Oberfläche
- 10: äußere Oberfläche
- 12: Deckel/Bodenelement
- 14: Positioniereinrichtung
- 16: Eingriffselement
- 18: Halteelement
- 20: Kopplungseinrichtung
- 23: Mediendrehverteiler
- 24a, 24b: Kommunikationswege
- 24: Sensor
- 28: Temperiermittel
- 30: Temperiermittel (alternativ/optional)
- 31: weiteres Temperiermittel (elektrisch und/oder optisch)
- 32: Umschaltventilgruppe

## Patentansprüche

1. Verfahren zum Behandeln von Einrichtungen einer Anlage zum Herstellen von Kunststoffbehältnissen,
mit den Schritten:
- Auswählen mindestens einer Behandlungseinrichtung (1),
- Beaufschlagen der Behandlungseinrichtung (1) mit einer Sterilisiertemperaturzum Sterilisieren der Behandlungseinrichtung (1),
- Zuführen von mindestens einem Kunststoffvorformling zu der Behandlungseinrichtung,
- Umformen des Kunststoffvorformlings zu einem Kunststoffbehältnis,
- Abführen des mindestens einen Kunststoffbehältnisses aus der Behandlungseinrichtung (1).

2. Verfahren nach Anspruch 1,
**dadurch gekennzeichnet, dass**
die Behandlungseinrichtung (1) zumindest zeitweise zumindest teilweise ein Kunststoffbehältnis umschließt.

3. Verfahren nach Anspruch 1,
**dadurch gekennzeichnet, dass**
zumindest eine Blasform (1), eine Blasdüse, eine Reckstange, eine Bodenform und/oder eine auf dem Formträger angeordnete Klammer zum Übernehmen des Vorformlings aus einer Transfereinrichtung für die anschließende Sterilisierung als Behandlungseinrichtung (1) ausgewählt wird.

4. Verfahren nach einem der vorangegangenen Ansprüche,
**dadurch gekennzeichnet, dass**
mindestens ein permanent mit der Behandlungseinrichtung (1) in thermischer Verbindung stehendes Temperierungsmittel (6a-6d) vorgesehen ist, durch das die Behandlungseinrichtung (1) auf die Sterilisiertemperatur temperiert wird.

5. Verfahren nach Anspruch 4,
**dadurch gekennzeichnet, dass**
die Behandlungseinrichtung (1) von einem durch das Temperierungsmittel (6a-6d) temperierten Fluid zumindest abschnittsweise um- und/oder durchströmt wird.

6. Verfahren nach Anspruch 5,
**dadurch gekennzeichnet, dass**
das Fluid zumindest teilweise aus einer Wasser-Glykol-Mischung, Thermoöl, Wasser und/oder ähnlichem besteht.

7. Verfahren nach Anspruch 4,
**dadurch gekennzeichnet, dass**
die Behandlungseinrichtung (1) auf eine Sterilisiertemperatur von mindestens 60°C, bevorzugt 100°C und besonders bevorzugt mehr als 121°C temperiert wird.

8. Verfahren nach Anspruch 7,
**dadurch gekennzeichnet, dass**
die Sterilisiertemperatur durch elektrische Erwärmung oder durch elektromagnetische Erwärmung, insbesondere mittels Induktion, erreicht wird.

9. Verfahren nach Anspruch 1,
**dadurch gekennzeichnet, dass**
die Behandlungseinrichtung (1) nach dem Sterilisieren zum Zuführen von zumindest einem Behältnis geöffnet wird.

10. Verfahren nach Anspruch 1,
**dadurch gekennzeichnet, dass**
der Behandlungseinrichtung (1) vor und/oder während der Beaufschlagung mit der Sterilisiertemperatur mindestens ein Desinfektionsmittel zugeführt wird.

11. Verfahren nach Anspruch 1,
**dadurch gekennzeichnet, dass**
es sich bei der mindestens einen Behandlungseinrichtung (1), insbesondere um die Blasform (1), eine Blasdüse, eine Reckstange und/oder eine Bodenform handelt, welche mit Sterilisiertemperatur beaufschlagt werden.

12. Blasform (1) zum Umformen von Vorformlingen in Behältnisse, mindestens umfassend einen ersten Teil (2) einer Blasformwandung und einen gegenüber dem ersten Teil (2) bewegbaren zweiten Teil (4) einer Blasformwandung mit jeweils einer inneren und einer äußeren Oberfläche (8, 10), wobei durch die innere Oberfläche (8) zumindest abschnittsweise eine negative Behälterform ausgebildet ist und mittels einer Kopplungseinrichtung die Teile der Blasformwandung zum Ausbilden eines geschlossenen Zustands miteinander koppelbar sind,
**dadurch gekennzeichnet, dass**
mindestens im Bereich eines Teils (2, 4) der Blasformwandung zumindest zeitweise ein Temperierungsmittel (6a-6d) zum Temperieren der Blasform (1) mit einer Sterilisiertemperatur angeordnet ist.

13. Blasform (1) nach Anspruch 13,
**dadurch gekennzeichnet, dass**
eine Steuerungsvorrichtung (26) vorhanden ist, die zum Steuern des Temperiermittels (6a-6d, 28, 30, 31) zum Temperieren der Blasform (1) ausgebildet ist, wobei die Blasform (1) außerhalb des eigentlichen Betriebs auf eine bestimmte Sterilisiertemperatur bringbar ist.

14. Blasform (1) nach Anspruch 13,
**dadurch gekennzeichnet, dass**
die Sterilisiertemperatur durch eine der Blasform zugeordnete Heizspirale oder Spule erzeugt wird.

15. Verwendung eines Temperierungsmittels (6a-6d) zum Temperieren einer Blasform (1) einer Behälterbehandlungsanlage mit einer Sterilisiertemperatur.
